Europäisches Patentamt

European Patent Office

Office européen des brevets

(19)

(11) Publication number: **0 354 624**
**A2**

## EUROPEAN PATENT APPLICATION

(21) Application number: 89202063.7

(51) Int. Cl.⁴ **C12N 15/00 , C12N 1/20**

(22) Date of filing: 09.08.89

The applicant has filed a statement in accordance with Rule 28 (4) EPC (issue of a sample only to an expert). Accession number(s) of the deposit(s): CBS 512.88.

(30) Priority: 11.08.88 EP 88201714
28.09.88 EP 88202118
03.03.89 EP 89200522

(43) Date of publication of application:
**14.02.90 Bulletin 90/07**

(84) Designated Contracting States:
**AT BE CH DE ES FR GB GR IT LI LU NL SE**

(71) Applicant: **GIST-BROCADES N.V.**
**Wateringseweg 1**
**NL-2611 XT Delft(NL)**

(72) Inventor: **Groenen, Martien A. M.**
**Marshallstraat 60**
**NL-6671 BK Zetten(NL)**
Inventor: **Veenstra, Annemarie E.**
**Nierop 27**
**NL-2151 XH Nieuw Vennep(NL)**
Inventor: **Van Solingen, Pieter**
**Rossini 16**
**NL-2671 VZ Naaldwijk(NL)**
Inventor: **Koekman, Bertus Pieter**
**Prunusstraat 8**
**NL-2636 BG Schipluiden(NL)**

(74) Representative: **Matulewicz, Emil Rudolf**
**Antonius, Dr. et al**
**Gist-Brocades NV Patents and Trademarks**
**Department Wateringseweg 1 P.O. Box 1**
**NL-2600 MA Delft(NL)**

(54) **A method for identifying and using biosynthetic or regulatory genes for enhanced production of secondary metabolites.**

(57) Novel methods and compositions are provided for the enhanced production of secondary metabolites. Methods are provided for identifying sequences which when transformed into secondary metabolite producing hosts, enhance the production of secondary metabolite. The process is exemplified for penicillin. In addition, the P. chrysogenum acyltransferase gene has been isolated and sequenced.

# A METHOD FOR IDENTIFYING AND USING BIOSYNTHETIC OR REGULATORY GENES FOR ENHANCED PRODUCTION OF SECONDARY METABOLITES

INTRODUCTION

Technical Field

The subject field concerns the isolation and use of genes for the production of secondary metabolites.

Background and Relevant Literature

As a result of classical strain improvements, penicillin production has increased enormously over the last four decades. These classical strain improvements were primarily based on random mutagenic treatments of Penicillium chrysogenum and subsequent selection for mutants that produced more penicillin. The development of cloning techniques however has added a potentially powerful new tool to further improve penicillin production in this fungus.

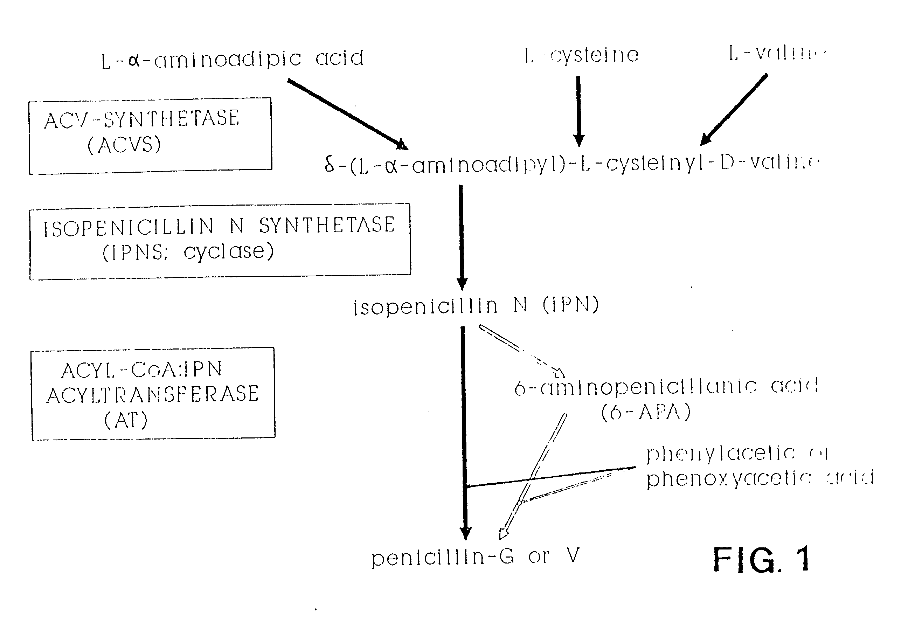

Penicillin is produced by the filamentous fungus P. chrysogenum in several enzymatic steps (e.g. E. Alvarez et al., Antimicrob. Agents Chemother. 31 (1987) pp. 1675-1682). These steps are shown in Figure 1. Throughout this specification is meant by genes directly involved in the biosynthetic pathway, those genes that encode the enzymes active in the several steps leading to the production of a secondary metabolite. So in case of the production of penicillin G or V, the genes encoding the enzymes in Figure 1 are meant. The first reaction is the formation of the tripeptide δ-(L-α-aminoadipyl)-L-cysteinyl-D-valine from α-amino adipic acid, cysteine and valine. The enzyme that is responsible for this reaction is the ACV synthetase (hereinafter referred to as ACVS), a large multifunctional enzyme. The tripeptide is cyclised by the action of the isopenicillin N synthetase (hereinafter referred to as IPNS) or cyclase. The reaction product is is isopenicillin N, a compound that contains the typical $\beta$-lactam ring structure and that possesses antibacterial activity. The final step in the formation of penicillin is the exchange of the α-aminoadipic acid side chain of isopenicillin N by a more hydrophobic side chain. The hydrophobic side chains commonly used in industrial production are either phenylacetic acid, yielding penicillin G and phenoxyacetic acid, yielding penicillin V. The side chain exchange has been proposed to be a reaction catalysed by a single enzyme (A.L. Demain (1983) in: A.L. Demain and N.A. Solomon (ed), Antibiotics containing the $\beta$-lactam structure I. Springer Verlag, Berlin; pp. 189-228). However, a two step reaction involving 6-APA as an intermediate is also possible (E. Alvarez et al., vide supra). The enzyme that has been identified to be involved in the final reaction is the acylCoA:6-APA acyltransferase (hereinafter referred to as AT); this enzyme has been purified to homogeneity (E. Alvarez et al., vide supra). The involvement of a second enzyme, catalysing the reaction from IPN to 6-APA, cannot yet be confirmed nor excluded.

It is not clear either whether one or more enzymatic reactions are rate limiting in the process of penicillin biosynthesis, and if so, which enzymatic steps are involved.

Since the penicillin biosynthetic route begins with three amino acids, which each in their turn are part of other metabolic routes, regulatory steps in these routes will also influence the biosynthesis of penicillin. On the other hand, the production of penicillin is subject to a complex mechanism of carbon catabolite repression and nitrogen source control (J.F. Martin et al. In: H. Kleinkauf, H. von Döhren, H. Donnauer and G. Nesemann (eds), Regulation of secondary metabolite formation. VCH Verlaggesellschaft, Weinheim (1985), pp. 41-75). Regulatory proteins may also be involved in these types of regulation. These regulatory proteins, and the proteins regulated by them, are defined to be indirectly involved in the biosynthetic pathway of a secondary metabolite, in this case penicillin.

Until recently, the gene of only one of the enzymes active in the biosynthetic pathway to penicillin G, the isopenicillin N synthetase (IPNS) or cyclase, had been cloned and sequenced (L.G. Carr et al., Gene 48 (1986) pp. 257-266), using the corresponding Acremonium chrysogenum gene (S.M. Samson et al. Nature 318 (1985) pp. 191-194). The latter gene was cloned and identified by purifying the IPNS protein, determining the aminoterminal amino acid sequence, preparing a set of synthetic oligodeoxyribonucleotides according to this sequence and probing a cosmid genomic library with these mixed oligodeoxyribonucleotides (S.M. Samson, vide supra).

Strain improvement studies using the cloned Penicillium chrysogenum isopenicillin N synthetase genes

2

in Penicillium chrysogenum resulted in enhanced enzyme activity, but no improvement in penicillin production, nor stimulation of penicillin synthesis is found (P.L. Skatrud et al., Poster presentation 1987 Annual meeting of Society of Industrial Microbiology, Baltimore, August 1987, abstract published in SIM News 37 (1987) pp. 77).

It has been documented that the biosynthesis of $\beta$-lactam antibiotics is subject to glucose repression (J.F. Martin and P. Liras, TIBS 3. (1985), pp. 39-44). This repression by glucose has been unequivocally established for the formation of the tripeptide by the ACVS and for the activity of the IPNS (Revilla et al., J. Bact. 168 (1986), pp. 947-952). For acyltransferase, on the other hand, the data are less convincing. Revilla et al (vide supra) report that AT is not subjected to glucose repression, but other data suggest that AT activity is absent, or at least decreased, in the presence of glucose (B. Spencer and T. Maung, Proc. Biochem. Soc. 1970, pp. 29-30).

It is unknown at which stage of the expression the repression by glucose is exerted; this can be at the transcriptional or at the translational level. If the former regulation applies, differences in mRNA levels between producing and non-producing cultures could be employed to isolate the said genes.

There is further uncertainty on the levels of the mRNA's encoding the various enzymes in penicillin producing cells.

## SUMMARY OF THE INVENTION

Subtraction isolation methods are employed for identifying genes associated with the production of secondary metabolites in microorganisms. The method is exemplified with production of penicillin in P. chrysogenum.

## BRIEF DESCRIPTION OF THE DRAWINGS.

Figure 1
The biosynthetic pathway to penicillin G or V in P. chrysogenum is shown schematically.

Figure 2
Physical map of lambda clones isolated by the method of the invention. Clones lambda G2 and lambda B21 contain the cyclase and acyltransferase gene cluster. Clones lambda B9, G5 and L5 contain the cryptic gene Y. Other lambda clones contain other cryptic genes.
E = EcoRI; B = BamHI; H = HindIII; K = KpnI;
S = SalI; Sa = SacI; Sp = SphI; P = PstI; X = XhoI;
Xb = XbaI; Hp = HpaI; N = NcoI and Bg = BglII

```
⬚⬚⬚⬚⬚ = right arm of bacteriophage lambda EMBL3 (9 kb)
████████ = left arm of bacteriophage lambda EMBL3 (20.3 kb)
- - - - - - - = region that hybridizes to pen⁺ cDNA
- - - - - - - = unclear region of map
(   )      = position/occurrence of restriction site not
clear
```

la and ra are left arm and right arm respectively of bacteriophage lambda.

Figure 3
Nucleotide sequence and deduced amino acid sequence of the P. chrysogenum acyltransferase gene.

Figure 4
Nucleotide sequence and deduced amino acid sequence of the P. chrysogenum pyrG gene. This sequence forms the major part of the 2.4 kb EcoRI fragment that acts as a transformation stimulating sequence.

Figure 5
Nucleotide sequence of the promoter of the P. chrysogenum phosphoglycerate kinase gene.

Figure 6

A restriction site and functional map of pUC13::pyrG.

Figure 7

A restriction site and functional map of pPS54.

Figure 8

A restriction site and functional map of pRH05.

Figure 9

A restriction site and functional map of PGJ01 A and B.

Figure 10

A restriction site and functional map of pPS47.

## DESCRIPTION OF THE SPECIFIC EMBODIMENTS

In accordance with the subject invention, DNA fragments are identified which include sequences which are mono- or polycistronic. The gene(s) encoded by the sequences are translated to enzymes concerned with the production of secondary metabolites or other products of commercial interest. These sequences of interest are identified by comparison of RNA sequences isolated from an organism competent to produce the secondary metabolite, where the genes of interest are actively expressed, and a microorganism in which expression is silent. In this way DNA fragments are provided encoding one or more genes that are differentially expressed and that are involved in the formation of a product of commercial interest. Differentially expressed is used throughout this application for expression of the gene(s) of interest that is specifically active only under certain defined conditions and that is absent (which is meant in this specification to be present at a low level e.g. a level of 5% or less, as compared to the active stage) under other, equally well defined conditions. The absence of expression may be a result of repression or lack of induction of gene expression, mutation, or other events which result in transcriptional silence of the gene(s) of interest. The DNA which is isolated may result from screening a gene library, either genomic or cDNA library contained in e.g. a lambda or a cosmid cloning vector or in an expression vector. By employing a cDNA probe enriched for sequences expressed during the biosynthesis of secondary metabolites, positive hybrids may be identified in the library for subsequent manipulation to provide for expression constructs for the enzyme(s) associated with the production of the secondary metabolite. Therefore a gene library of a microorganism is screened using two cDNA probes, one of which is enriched for sequences from the transcriptionally active state and the other is derived from the transcriptionally silent situation. By comparison and subtraction those clones that contain gene(s) that are actively expressed under the defined active conditions only, can be isolated.

The method is exemplified by the isolation of genes involved in the biosynthesis of a secondary metabolite, more specifically penicillin, using two cDNA probes, from lactose grown (producing) and glucose grown (non-producing) mycelium.

The identified DNA sequences will comprise at least one gene encoding an antibiotic biosynthetic enzyme and/or a regulatory protein from the entire biosynthetic pathway, or more generally any protein that is involved in whatever way, either positive or negative, in the biosynthesis of said antibiotic.

The positively acting constructs, when properly introduced into a suitable host microorganism increase the efficiency of the biosynthetic pathways operative in β-lactam producing microorganisms by increased gene dosage, or by higher gene expression. On the other hand, constructs may be isolated that have a negative effect on the antibiotic production (e.g. formation of side products). These constructs are employed to inactivate the negatively acting gene by gene replacement or other methods with a similar effect. Both uses result in higher yields of the desired antibiotic during industrial production. This method is exemplified by and finds particular application with β-lactam producing microorganisms for the production of antibiotics, particularly penicillins. Preferably, the expression cassette will include genes encoding enzymes that catalyze rate-limiting steps or genes encoding regulatory proteins for induction of transcription or otherwise.

The subject method further provides sequences for which the encoded product is not known, but the sequence is found to provide an enhanced yield of a desired product. These sequences are referred to as "cryptic genes", which means sequences obtainable by isolation methods described herein, which sequences encompass genes for which no known function is yet assignable. These genes are characterized by being dosed and/or expressed in higher amounts in the transformed host-microorganisms as compared with their untransformed hosts. In addition to the "cryptic genes", other genes provided are IPNS and acyltransferase. A cryptic gene named "Y" was shown to provide enhanced biosynthesis of penicillin.

The microorganisms employed in the subject invention include both prokaryotes and eukaryotes, including bacteria such as those belonging to the taxonomic group of the Actinomycetes or Flavobacterium,

4

or fungi including yeasts, belonging to the genera Aspergillus, Acremonium or Penicillium.

Depending upon the source of the fragment, either genomic or cDNA, either prokaryotic or eukaryotic, various expression cassettes may be constructed. With genomic DNA from a bacterium, the fragment containing a mono- or polycistronic coding region may include its own transcriptional initiation regulatory region, as well as a transcriptional termination region and appropriate translational signals, e.g. Shine-Delgarno sequence and stop codons. Where the genomic DNA is from a fungus, normally only one gene will be associated with a transcriptional initiation regulatory region, so that each gene will have its own independent transcriptional initiation regulatory region. Where cDNA is employed, it will be necessary to provide an appropriate transcriptional initiation regulatory region, depending on the host organism used for subsequent expression.

The genes of interest may be obtained by screening a DNA library prepared from said microorganism with probes obtained from mRNA or DNA derived from a first culture of said microorganism, producing said secondary metabolite; screening said DNA library with probes obtained from mRNA or DNA derived from a second culture of said microorganism or a mutant thereof, lacking the production of said secondary metabolite; and identifying fragments comprising genes transcribed in said first culture which are not substantially transcribed in said second culture.

Particularly valuable genes include those which are specifically expressed during antibiotic biosynthesis, including the genes encoding $\beta$-lactam biosynthetic enzymes known in the art, e.g. tripeptide synthetase (ACVS), cyclase (IPNS), acyltransferase (AT), epimerase, expandase, hydroxylase, transcarbamoylase, methoxylase, transacetylase. Preferably genes encoding IPN:6-APA acyltransferase or the cryptic gene "Y" are dosed or expressed in higher amounts resulting in higher yields of the desired antibiotic in the transformed fungus.

It will be appreciated by those skilled in the art, that the gene(s) to be expressed in a $\beta$-lactam producing host may either carry its own native promoter sequence which is recognized by an RNA polymerase of the host cell, or may be ligated to any other suitable promoter, e.g. that of a different $\beta$-lactam biosynthetic gene or that of a glycolytic gene such as phosphoglycerate kinase, glyceraldehyde phosphate dehydrogenase, triose phosphate isomerase, or that of the translational elongation factor, Ef-Tu, or the like.

Such a promoter may be employed to influence regulation of expression of one or more genes encoding said enzymes. This will lead to an increased production of the antibiotic after transformation, since penicillin production is now also possible under conditions that in the untransformed host strain do not lead to penicillin production, e.g. glycolytic enzymes are expressed in the presence of glucose, while the production of penicillin, on the other hand, is repressed in the presence of glucose (J.F. Martin, vide supra). By bringing the expression of penicillin biosynthetic genes under the control of a promoter of a glycolytic gene, the genes can also be expressed in the presence of glucose and hence penicillin can be produced early in the fermentation, when a high concentration of glucose is required for the generation of a sufficient amount of mycelium. Also the selection marker can be brought under control of such a promoter.

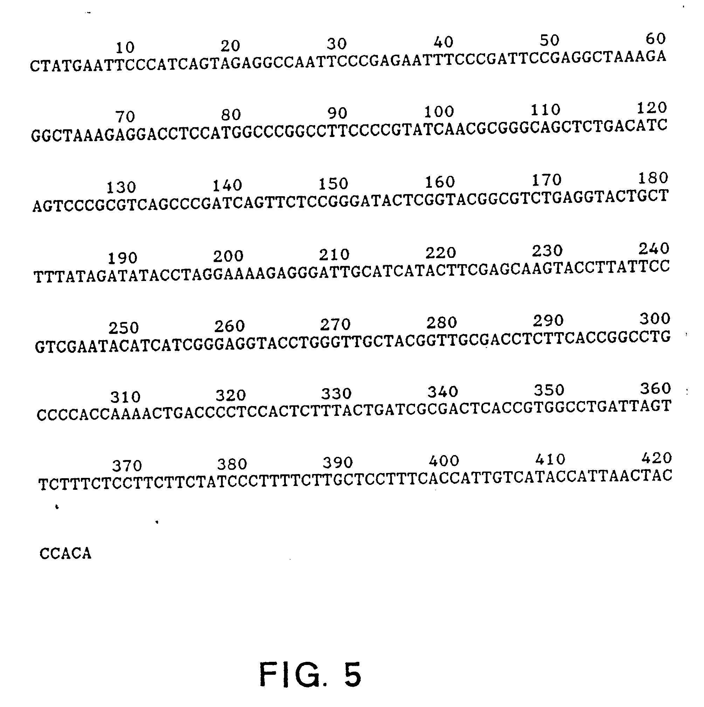

The present invention exemplifies the promoter of the phosphoglyceratekinase gene of P. chrysogenum as a promoter to be used to overcome glucose repression of penicillin biosynthesis. This promoter was isolated from a genomic library of P. chrysogenum by standard methods, using oligodeoxyribonucleotide probes derived from the published yeast sequence of R.A. Hitzeman et al., Nucleic Acids Res. 10 (1982) pp. 7791-7808. The nucleotide sequence of the phosphoglyceratekinase promoter is specified in Figure 5.

For transformation of Penicillium constructs are employed including at least one marker for selection of transformed cells and, preferably, for enhancing maintenance of the integrated DNA. Therefore, the vector preferably includes a DNA sequence known to enhance transformation efficiencies. An example of such a DNA sequence is the "ans"-element, isolated from Aspergillus nidulans (cf. Ballance and Turner, Gene 36 - (1985) pp. 321-331). The present invention provides a DNA sequence, isolated from the genome of P. chrysogenum, that has been identified as a sequence with an effect similar to the effect of the "ans" sequence. Since this sequence is native to P. chrysogenum, it can be used to increase transformation efficiencies in P. chrysogenum. The DNA sequence encompasses the P. chrysogenum pyrG gene and can be used either alone, in combination with a pyrG-host, in which case said DNA sequence provides both the selection for transformants and the transformation enhancing effect (cf. EP-A-260762), or in combination with another selection marker, e.g. a gene encoding resistance to a biocide, such as phleomycin. In the latter case selection for transformants and the transformation enhancing effect are provided by two separate DNA sequences and the sole function of the pyrG element is to enhance transformation frequencies.

Useful markers for the selection of transformant clones may be homologous or heterologous biosynthetic genes capable of complementing an auxotrophic requirement of the host cell, caused by a defect in a metabolic route to an amino acid, e.g. arginine, a nucleotide precursor, e.g. uracil, and the like.

5

The structural gene providing the marker for selection may be native to the wild-type Penicillium host or a heterologous structural gene which is functional in the host. For example, structural genes coding for an enzyme in a metabolic pathway may be derived from Penicillium or from other filamentous fungi, e.g. Aspergillus, Neurospora, Podospora, or yeasts, where the structural gene is functional in the Penicillium host and complements the auxotrophy to prototrophy.

The complementing structural gene may be derived from a metabolic pathway, such as the synthesis of purines or pyrimidines (nucleosides) or amino acids. Of particular interest are structural genes encoding enzymes in the pyrimidine pathway, e.g. the gene encoding the enzyme orotidine-5'-phosphate decarboxylase (pyrG or pyr4). Other genes of interest are amino acid biosynthetic genes, e.g. ornithine carbamoyl transferase (argB) and arginino-succinate lyase (arg4). The use of the above-mentioned selection markers is provided in EP-A-260762.

Instead of auxotrophic markers, fermentation markers may be used, such as the capability of using amides as a sole source of carbon or nitrogen, growth on various sugars, e.g. galactose or the like.

Furthermore, genes encoding resistance to biocides may be used, such as hygromycin, gentamicin, phleomycin, glyphosate, bialaphos, and the like.

Constructs will be provided comprising the sequence of interest, and may include other functions, such as replication systems in one or more hosts, e.g. cloning hosts and/or the target host for expression of the secondary metabolite; one or more markers for selection in one or more hosts, as indicated above; genes which enhance transformation efficiency; or other specialized function.

The construct will contain at least one gene isolated by the method of this invention. The construct may be prepared in conventional ways, by isolating other desired genes from an appropriate host, by synthesizing all or a portion of the genes, or combinations thereof. Similarly, the regulatory signals, the transcriptional and translational initiation and termination regions, may be isolated from a natural source, be synthesized, or combinations thereof. The various fragments may be subjected to endonuclease digestion (restriction), ligation, sequencing, in vitro mutagenesis, primer repair, or the like. The various manipulations are well known in the literature and will be employed to achieve specific purposes.

The various fragments may be combined, cloned, isolated and sequenced in accordance with conventional ways. After each manipulation, the DNA fragment or combination of fragments may be inserted into the cloning vector, the vector transformed into a cloning host, e.g. E. coli, the cloning host grown up, lysed, the plasmid isolated and the fragment analyzed by restriction analysis, sequencing, combinations thereof, or the like. E. coli may also be used as a host for expression of the genes of interest, with the aim to produce high amounts of protein.

Various vectors may be employed during the course of development of the construct and transformation of the host cell. These vectors may include cloning vectors, expression vectors, and vectors providing for integration into the host or the use of bare DNA for transformation and integration.

The cloning vector will be characterized, for the most part, by a marker for selection of a host containing the cloning vector and optionally a transformation stimulating sequence, may have one or more polylinkers, or additional sequences for insertion, selection, manipulation, ease of sequencing, excision, or the like.

Expression vectors will usually provide for insertion of a construct which includes the transcriptional and translational initiation region and termination regions; alternatively the construct may lack one or both of the regulatory regions, which will be provided by the expression vector upon insertion of the sequence encoding the protein product.

The DNA encoding enzyme(s) of interest may be introduced into a Penicillium host in substantial accordance with the procedure as described in EP-A-260762.

Efficient transformation of Penicillium is provided to produce transformants having one or more structural genes capable of expression, particularly integrated into the host genome (integrants). DNA constructs are prepared which allow selection of transformed host cells. Conditions are employed for transformation which result in a high frequency of transformation, so as to ensure selection and isolation of transformed hosts expressing the structural gene(s) of interest. The resulting transformants provide for stable maintenance and expression of the integrated DNA. It will be appreciated that the transformed host according to the invention can be used as starting strain in strain improvement processes other than DNA mediated transformation, for instance, protoplast fusion, mass mating and mutation. The resulting strains are considered to form part of the invention.

The genes of interest to be introduced by transformation may form an integral part of the transformation vector, but it will often be more convenient to offer these genes on a separate vector in the transformation mixture, thus introducing the said genes by cotransformation along with the selective vector, which is a fairly efficient process in filamentous fungi (P.J. Punt et al., Gene 56 (1987) pp. 117-124; K. Wernars et al, Mol. Gen. Genet. 209 (1987) pp. 71 77; I.E. Mattern et al., Mol. Gen. Genet. 210 (1987) pp. 460-461).

As a result of the transformation, there will be at least one copy of the gene(s) of interest frequently two or more, usually not exceeding about 100, more usually not exceeding about 10. The number will depend upon whether integration or stable episomal maintenance is employed, the number of copies integrated, whether the subject constructs are subjected to amplification and the like.

The subject invention exemplifies a method to isolate genes involved in penicillin biosynthesis from a gene library of the producing organism, P. chrysogenum using specific cDNA probes which method is paradigmatic for identifying cryptic genes for the enhanced production of secondary metabolites. This method for the isolation of DNA constructs encoding one or more genes that take part in the biosynthesis of secondary metabolites, comprises the screening of a gene library with a cDNA probe enriched for sequences specifically expressed during the biosynthesis. By "specifically" expressed is meant that expression of these genes is silent or at a very low level (for example less than 5% of production level) in the absence of penicillin production and in contrast is high during penicillin production. To this end, radioactive or other labeled cDNA probes are synthesized on mRNA templates isolated from P. chrysogenum mycelia during the penicillin production phase.

The probes are then enriched for sequences hybridizing specifically to the desired genes by eliminating those cDNA sequences that hybridize to mRNA derived from a Penicillium fermentation under conditions not allowing penicillin production. e.g. high glucose concentration. Using this enriched probe, clones are selected from a P. chrysogenum gene library that do not hybridize to a probe derived from non-producing mycelia. A large number of the clones thus isolated appears to encode the penicillin biosynthetic enzyme isopenicillin N synthetase (IPNS or cyclase).

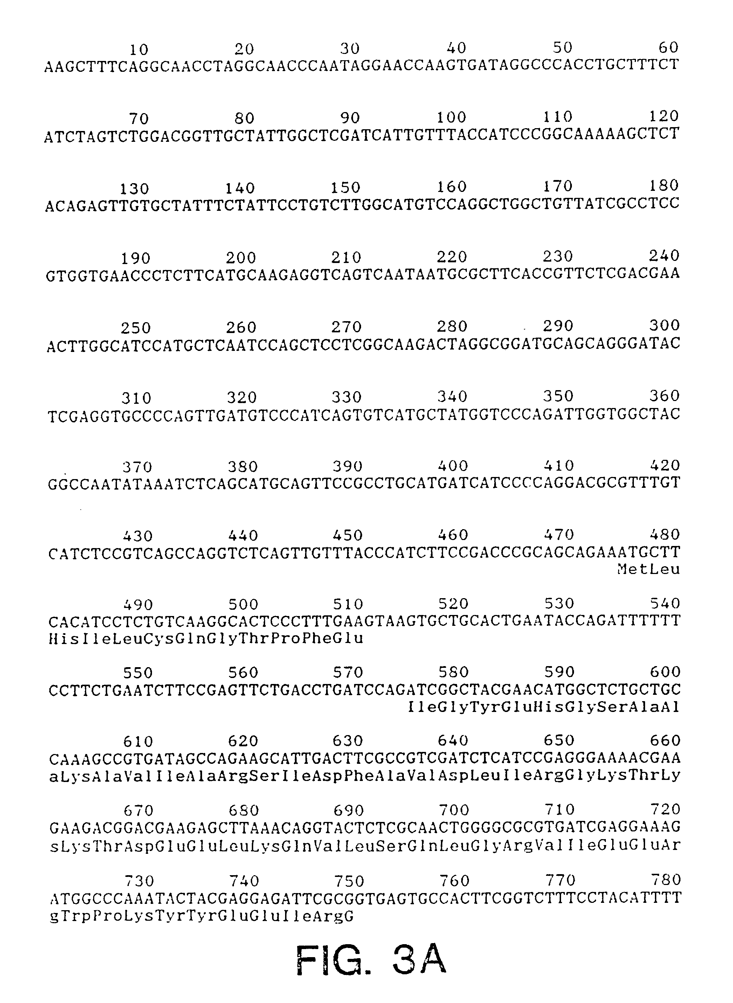

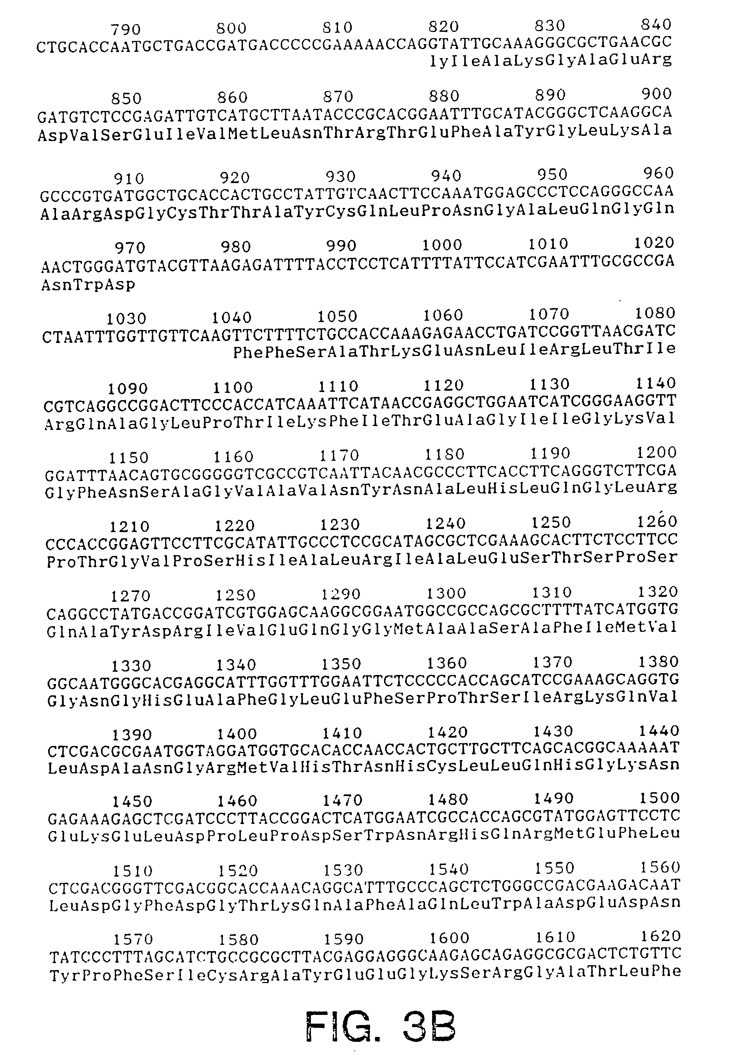

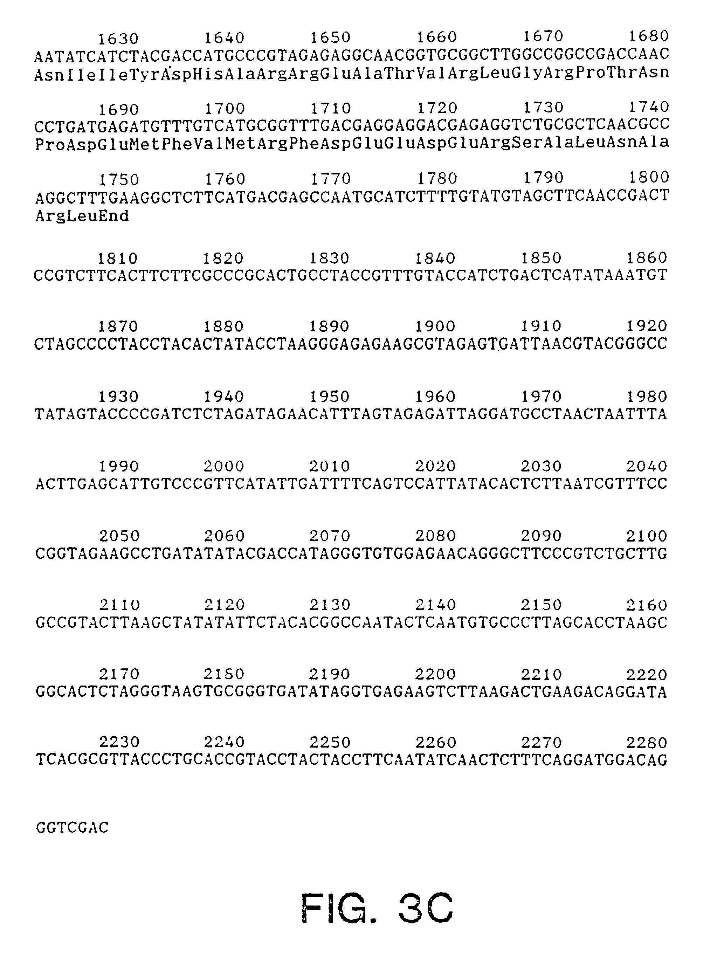

Furthermore, among the selected clones, several copies of the gene encoding the side-chain exchanging enzyme (acyltransferase) are found to be present. This was proven with experiments where a DNA probe was employed, based on the aminoterminal peptide sequence of the purified enzyme. The identity of these clones is biochemically and biologically verified. The nucleotide and deduced amino acid sequence of the acyltransferase gene are specified in Figure 3. Surprisingly, the genes encoding the isopenicillin N synthetase and acyltransferase enzymes are present together on one DNA fragment. This was demonstrated by hybridization of a genomic library of P. chrysogenum in the lambda vector EMBL 3 with separate probes, specific for each of both said genes. Identical clones hybridize separately with both probes.

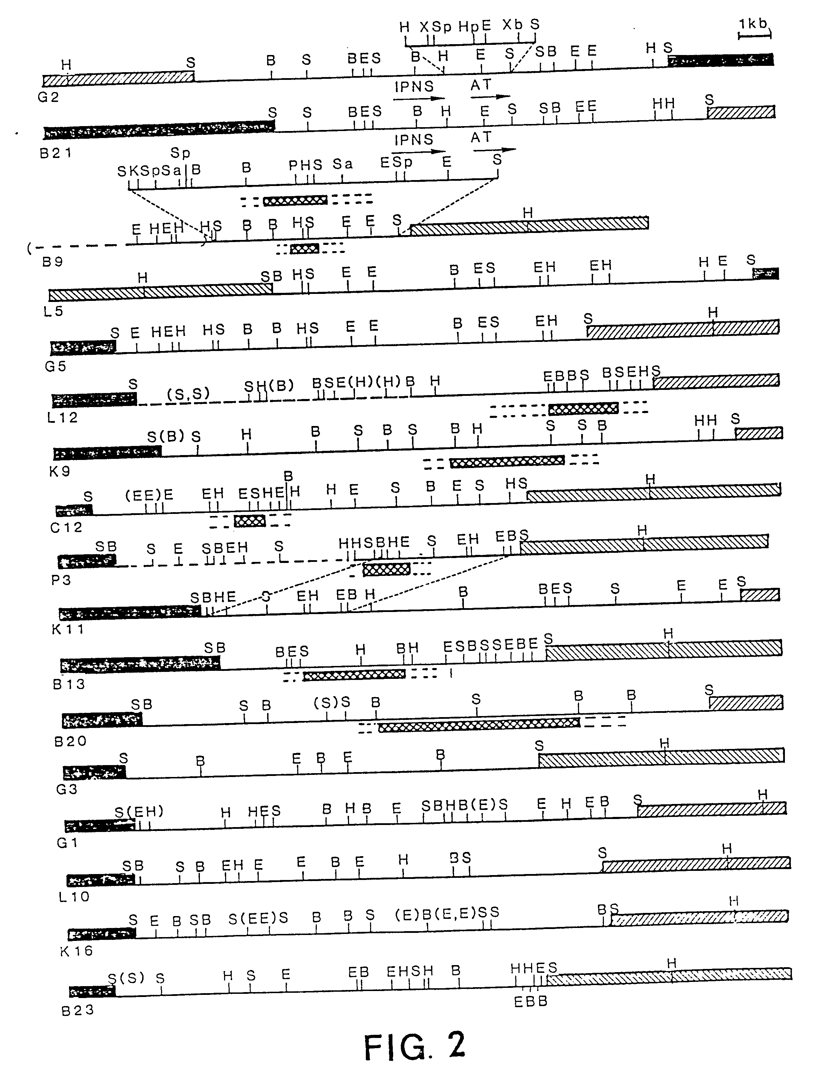

Moreover, after construction of a physical map of one genomic lambda clone, and hybridization of restriction digests of the lambda clone with separate probes for both of the genes, the genomic organization was shown to be such as depicted in Figure 2 (clones B21 and G2). The presence of both genes on one large DNA fragment allows construction of P. chrysogenum strains with a higher dosage of both the isopenicillin N synthetase and acyltransferase genes, without disturbing the relative organization or the balanced expression of both genes. Moreover, the introduction of multiple copies of said large DNA fragment allows expression of both genes on said DNA fragment in their natural environment with upstream and downstream sequences that are identical to the normal situation.

Both the balanced expression and the maintenance of the natural environment prove to be beneficial for the efficiency of gene expression and hence of penicillin production. The isolation techniques of the isopenicillin N-synthetase plus acyltransferase gene cluster may be advantageously applied for the isolation of other penicillin biosynthetic genes by chromosome walking techniques, where the penicillin biosynthetic genes may be clustered.

Furthermore, a number of cryptic genes have been isolated, to which no function has been assigned yet but which are likely to play a part in β-lactam biosynthesis. A physical map of the cryptic genes of the invention is provided in Figure 2 (clones B9-B23).

Of these "cryptic" genes, the gene designated Y, present on clones B9, L5 and G5, when transformed (on a 3.0 kb SphI + BamHI subfragment) to a suitable host, stimulates the production of penicillin by 26%, compared to the untransformed host. This demonstrates the involvement of the product of gene Y in penicillin biosynthesis. Moreover, this demonstrates that transformation using genes isolated by the method of the invention, without knowing their function or identity, can be applied successfully in strain improvement of P. chrysogenum.

The present invention is further exemplified by transforming Penicillium chrysogenum with genes that are specifically expressed under conditions where the antibiotic is synthesized, and which encode gene products catalyzing biosynthetic reactions leading to the said antibiotics.

One such enzyme, acyltransferase (hereinafter referred to as AT), catalyzes the final step in penicillin biosynthesis, i.e. the exchange of the aminoadipyl moiety of isopenicillin N with a hydrophobic acyl side chain precursor, e.g. phenylacetic or phenoxyacetic acid, thus yielding penicillin G or V, respectively.

The acyltransferase gene of P. chrysogenum is provided, including the nucleic acid sequence. The invention provides conservative mutations, where the sequence encodes the same amino acid sequence, but may have as many as 30% different bases, more usually not more than about 10% different bases, or mutations which are non-conservative, where fewer than about 10%, more usually fewer than about 5%, and preferably not more than about 1% of the amino acids are substituted or deleted, and there are fewer than 5% of inserted amino acids, where the percent is based on the number of naturally occurring amino acids. In addition, fragments of both the nucleic acid encoding the enzyme, usually at least about 9 codons, more usually at least about 15 codons may be employed, as well as their expression products, as probes, for the production of antibodies, or the like. The probes may be used to identify the enzyme in other species by employing the nucleic acids for hybridization or the antibodies for identification of cross-reactive proteins.

The search for unnatural side chain precursors or other penicillins or cephalosporins that will serve as a substrate for the acyltransferase can be complemented by a search for mutant acyltransferase enzymes that will accept as a substrate side chain precursors other than phenylacetic acid or phenoxyacetic acid, or penicillins or cephalosporins other than the natural substrate isopenicillin N. The present invention provides the starting material for such a search for a mutant acyltransferase enzyme. E. coli is the best host for mutational cloning experiments; since E. coli lacks the splicing machinery for the removal of the introns present in this acyltransferase gene, a cDNA clone of the acyltransferase gene is the sequence of choice for expression of the enzyme in E. coli. This cDNA sequence can be readily mutated by procedures well known in the art, such as, for example, treatment with radiation (X-ray or UV) or chemical mutagens (such as ethylmethanesulfonate, nitrosoguanidine or methylmethanesulfonate) or site specific mutagenesis, to obtain mutant enzymes that on the one hand recognize unnatural side chain precursors as a substrate and catalyze the formation of unnatural penicillins from isopenicillin N, or on the other hand catalyze a side chain exchange reaction on penicillins or cephalosporins, other than isopenicillin N.

The isolation of the AT-, Y- and other penicillin biosynthetic genes allows for the identification of regulatory elements of the individual genes such as a promoter, an upstream activating sequence (UAS), a terminator and the like. This can be achieved by sequence comparison of the genes amongst themselves and by comparison with the sequence as obtained for the isopenicillin N synthetase biosynthetic gene and other related genes. This latter comparison, moreover, may disclose the specific nature of the regulation of the gene expression of the group of penicillin biosynthetic genes.

Identification of such a "penicillin biosynthetic regulatory element" leads to identification of specific regulatory proteins by means of standard techniques as gel retardation, cross-linking, DNA footprinting and the like. Isolation of the specific regulatory protein by affinity chromatography will result in the cloning of the gene encoding said protein and subsequent manipulation in a suitable host.

By use of the cloned AT-gene, Y-gene and other penicillin biosynthetic genes, modified enzymes may be designed and synthesized. These modifications will result in modified characteristics of the enzymes, such as a change in pH or temperature optimum, a change in stability or a change in substrate specificity. Host strains, transformed with genes encoding these modified enzymes, may be programmed to perform antibiotic synthesis under different conditions or to synthesize alternative antibiotics, e.g. ampicillin instead of penicillin.

In another aspect of the invention, the cloned genes may be used to transform host strains that do not naturally possess these enzymes. It is known that Streptomyces and Acremonium do not possess the AT-enzyme, while on the other hand Penicillium lacks the genes from the cephalosporin and cephamycin biosynthetic enzymes. Introduction of such genes into the hosts will result in biosynthesis of cephalosporin or cephamycin by Penicillium or penicillin or cephalosporins with a hydrophobic side chain by Acremonium.

It is evident from the following results that secondary metabolite production can be greatly enhanced by employing screening procedures which allow for identification of DNA sequences associated with production of a secondary metabolite. By using subtraction methods in identifying specific sequences associated with secondary metabolite production, mRNA and cDNA may be isolated and identified for use as probes. Thus, fragments containing cryptic genes, which will not yet have a known function are found to greatly enhance secondary metabolite production and may be transformed into a host for production of the secondary metabolite. This procedure is specifically exemplified for penicillin.

In addition, an acyltransferase gene is provided which finds use in a variety of ways, as an enzyme for modifying $\beta$-lactam compounds, as a label, as a source of an antigen for a production of antibodies to acyltransferase, as a source for a promoter sequence, as a source to express high amounts of protein for crystallization as a template for in vitro mutagenesis to obtain an enzyme with modified characteristics, and the like.

All publications and patent applications cited in this specification are herein incorporated by reference as if each individual publication or patent application were specifically and individually indicated to be

8

incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

The following examples are offered by way of illustration and not by way of limitation.

## EXPERIMENTAL

## EXAMPLE 1

Construction of a genomic library of Penicillium chrysogenum.

A genomic library of Penicillium chrysogenum (ATCC 28089) was constructed in substantial accordance with methods known in the art (T. Maniatis et al., (1982), Molecular cloning, A Laboratory Manual, Cold Spring Harbor Laboratory, N.Y.). Chromosomal DNA was extracted from Penicillium chrysogenum by forming protoplasts from the mycelium as previously described in EP-A-260762.

The protoplasts were then lysed by diluting the isotonic (0.7 M KCl) suspension with four volumes of TES buffer (0.05 M Tris-HCl pH 8.0, 0.1 M EDTA, 0.15 M NaCl). To the lysate, 1% sodium lauryl sulphate was added and the mixture was incubated at 55°C for 30 min. After one extraction with phenol and two extractions with chloroform, the DNA was precipitated with ethanol, dried, and dissolved in TE buffer (10mM Tris, 1mM EDTA pH 8.0). The DNA solution was then treated with 100 μg/ml RNase at 37°C for 1 h and subsequently with 200 μg/ml proteinase K at 42°C for 1 h. The solution was extracted once with phenol and twice with chloroform. An equal volume of isopropanol was layered on top of the aqueous phase and the DNA was collected at the interface by spooling around a glass rod. After drying, the DNA was dissolved in TE buffer. The molecular weight of the DNA preparation thus obtained was about $10^8$. The DNA was partially digested with Sau3A, ligated to dephosphorylated EMBL 3 arms cut with BamHI (Promega Biotec, Madison WI, USA), and packaged into bacteriophage lambda capsids using the Packagene System of Promega Biotec. All reactions were carried out in accordance with the manufacturer's recommendations except that the packaging reaction was carried out at 22°C for 2-3 hours. Libraries were amplified by plating the packaged phages, incubating for 7-8 hours at 37°C and eluting the phages using 4 ml of SM buffer (0.1 M NaCl, 0.01 M MgSO₄, 0.05 M Tris HCl pH 7.5, 0.01% gelatin) per Petri plate.

## EXAMPLE 2

Isolation of genes specifically expressed during penicillin biosynthesis using a differential screening procedure.

Genes that are specifically or predominantly expressed during penicillin biosynthesis were identified by probing the genomic library of Example 1 with labelled cDNA probes synthesized on mRNA templates extracted from producing (lactose-grown) and non-producing (glucose-grown) mycelia, and selecting the clones that gave predominantly a positive signal with the former (+) probe.

Messenger RNAs were isolated from cultures grown 3 or 6 days in the production medium (cf. Example 3) (+ preparation) or in the same medium with the lactose replaced by glucose (-preparation). The mycelia were collected by filtration, frozen in liquid nitrogen, homogenized and the mRNA isolated using the guanidinium isothiocyanate method as described by T. Maniatis et al. (vide supra).

cDNAS were synthesized and labelled to a high specific activity with [α-$^{32}$P] dATP against both mRNA populations in a reaction mixture of 30 μl containing
12.5 mM MgCl₂

9

50 mM Tris-HCl pH 8.3
100 mM KCl
125 mM DTT
2 u/μl RNasin
500 μM dGTP
500 μM dCTP
500 μM dTTP
25 μM dATP
0.1 μg/ml BSA
100-200 μg/ml poly A⁺RNA
50-60 μg/ml oligo dT·₁₂₋₁₈
1.2 u.μl reverse transcriptase
1.67 μCi/μl [α-³²P] dATP

in which the PolyA⁺ RNA and oligo-dT were mixed separately, heated to 100° C for 1 minute, and cooled in ice water prior to adding to the reaction mixture After 1.5 hours incubation at 42° C, 5 μl of 1 mM dATP was added and the incubation continued for 30 min. Subsequently, the reaction mixture was made 20 mM in EDTA, 40 mM in NaOH (final volume 100 μl) and heated to 65° C. After 1 hour incubation, 5 μl 1 M Tris-HCl pH 8.3, 40 μl 0.1N HCl, 7 μg calf thymus DNA, 100 μl TES buffer (10 mM Tris, 1 mM EDTA, 1% SDS pH 7.5) and 200 μl 5 M ammonium acetate were added and the DNA was precipitated with 800 μl ethanol for 16 hours at -20° C.

The precipitate was collected by centrifugation, washed with 70% ethanol, dried, and dissolved in 32.5 μl of TE buffer (10 mM Tris, 1 mM EDTA pH 8.0). The (+) cDNA preparation was then enriched for sequences specifically expressed during penicillin biosynthesis by two successive rounds (cascades) of hybridization against a (-) mRNA preparation in a reaction mixture of 75 μl containing

32.5 μl (+) cDNA
10 μl (-) mRNA (1 μg/μl)
30 μl 1M NaPO₄ pH 6.8
1.5 μl 10% SDS
1 μl 0.5 M EDTA

After incubation for 16 hours at 68° C, 102 μl of water was added (final phosphate concentration 170 mM) and the mixture passed through an hydroxylapatite column equilibrated in 170 mM phosphate at 68° C. Under these conditions, double stranded nucleic acids bind to the column whereas single stranded nucleic acids are eluted. The eluate was collected, dialyzed against TE buffer for 1.5 hours, and ethanol precipitated after addition of 4 μg carrier (calf thymus) DNA. This procedure was repeated and the final unbound cDNA was directly used as a probe to screen a genomic library of the Penicillium strain as follows:

A sample of the amplified library of Example 1 was plated onto 5 Petri plates so as to contain approximately 1500 plaques per plate. The plaques were transferred in duplicate to Gene Screen Plus filters (New England Nuclear) according to the manufacturer's recommendations. One set of filters was probed with the labelled, enriched (+) cDNA preparation; the duplicate set was probed with the labelled (-) cDNA as a control.

Positive plaques were purified and subjected to a second screening. In this way, 96 plaques were selected that gave a positive signal predominantly with the (+) cDNA probe.

DNAs of recombinant phages that had given a strong or moderate signal in the initial screening were labelled with ³²P and used as probes to screen Northern blots of Penicillium RNAs isolated from producing and non-producing mycelia, in order to establish the levels of expression under both conditions. In this way the recombinant clones were divided into three groups:

Class 1 contains genes highly expressed during penicillin biosynthesis and is exemplified by clones *
G2 and B21
* B9, L5 and G5
* L12
* K9

Class 2 moderately expressed, exemplified by
* C12
* P3 and K11
* B13
* B20

Class 3 weakly expressed, exemplified by
* G3

˙ G1

˙ L10

˙ K16

˙ B23

Physical maps of these recombinant phages are shown in Figure 2. Clones G2 and B21 gave a positive hybridization signal when probed with an isopenicillin N synthetase-specific probe (S.M. Samson et al., vide supra). Surprisingly. the same clones appeared also to hybridize to an acyltransferase-specific probe (see Example 5).

EXAMPLE 3

Purification of acyltransferase.

Penicillium chrysogenum strain (ATCC 28089) was inoculated (at $2 \times 10^6$ conidia/ml) in a complex seed medium containing: corn steep liquor (20 g.l); distiller solubles (20 g/l); sucrose (20 g/l); CaCO₃ (5 g/l) (pH before sterilization 5.7). After 36 hours incubation at 25 °C. 250 rpm, the obtained culture was used to inoculate twenty volumes of complex production media containing: Corn steep solids (35 g/l); lactose (25 g.l); potassium phenylacetate (2.5 g.l); MgSO₄.7H₂O (3 g.l); KH₂PO₄ (7 g/l); corn oil (2.5 g/l); CaCO₃ (10 g.l). After continuation of the incubation for another 48 hours, the mycelium was collected by filtration and the filter cake washed four times with cold 0.15 M NaCl.

200 grams (wet weight) of mycelium were suspended in 700 ml of 0.05 M Tris-HCl buffer (pH 8) containing 5 mM dithiothreitol (hereinafter referred to as TD buffer) and disrupted in a Braun desintegrator (Braun. Melsungen. F.R.G.) using Ballotini glass beads (Sigma type V. diameter 450-500 μm) for periods of 30 s at intervals of 15 s with refrigeration in an ethanol.dry ice bath. The extract was then centrifuged for 30 min. at 20,000 x g. This and all following steps were carried out at 4 °C. To 640 ml of the extract. 27 ml of a 10% w/v protamine sulphate solution in 0.05 M Tris-HCl pH 8 was slowly added. After stirring for 45 minutes. the nucleic acid precipitate was removed by centrifugation at 20,000 x g and the supernatant fractionated by precipitation with ammonium sulfate while maintaining the pH of the solution at 8.0 during the ammonium sulfate additions. The fraction precipitating between 40% and 55% saturation was dissolved in TD buffer containing 1 M ammonium sulfate and applied to a phenylsepharose CL-4B column (1.8 x 16 cm) equilibrated with the same buffer. The column was washed with TD buffer at a flow of 5 ml/min until no more unbound proteins were released.

Then the acyltransferase was eluted from the column with 40% ethylene glycol in 0.05 M Tris-HCl pH 8.0.

The eluted fractions were assayed for acyltransferase activity by incubating at 25 °C in a reaction mixture containing 0.2 mM phenylacetylcoenzyme A, 0.2 mM 6-aminopenicillanic acid, 5 mM dithiothreitol, 0.1 M Tris-HCl pH 8.0 and enzyme extract in a final volume of 200 μl. After 10 minutes the reaction was stopped by adding 200 μl methanol. The samples were centrifuged at 5000 x g and the penicillin G was assayed in the supernatant by conventional microbiological or chromatographic methods.

The active fractions from the phenylsepharose column were pooled and applied to a DEAE-Séphacel column (1.5 x 20 cm) equilibrated with TD buffer and the acyltransferase activity was eluted with a linear (0 - 0.25 M) gradient of NaCl in TD buffer at a flow rate of 0.25 ml/min. The pooled active fractions were precipitated with 70% ammonium sulfate and the pellet dissolved in 3 ml of TD buffer and applied to a Sephadex G-75 (coarse) column (2.6 x 70 cm) equilibrated with TD buffer. The acyltransferase was eluted using TD buffer at a flow of 9 ml/h and collected in the late part of the eluted fractions as a symmetrical peak of protein corresponding to acyltransferase activity. The final purification was 258-fold.

EXAMPLE 4

Determination of the amino-terminal amino acid sequence of acytransferase and design of the corresponding oligonucleotide probe mixtures.

The enzyme preparation, obtained as described in Example 3 was run on an SDS-PAGE gel (U.K. Laemmli, Nature, 227 (1970) pp. 680 ff) (13% acrylamide, 50 mA). A 29 kD - band (about 10 μg of protein) was cut out of the SDS - gel and the protein was electrophoretically transferred onto a PCGM-2 membrane (polybrene impregnated glassfibre, Janssen, Beerse, Belgium), using a Multiphor II Nova blot unit (LKB; 0.8 mA/cm², 90 min; electrode buffer 5 mM sodium borate pH 8.0). After blotting, the PCGM - membrane was washed four times with 25 mM NaCl, 10 mM sodium borate, pH 8.0 and air dried.

The PCGM - adsorbed protein band thus obtained was analyzed for N-terminal amino acid sequence, using a gasphase sequenator (Applied Biosystems model 470 a). The following sequence was determined:

thr-thr-ala-tyr-cys-gln-leu-pro-asn-gly-ala-leu-gln-gly-gln-asn-trp-asp

According to the underlined part of this amino acid sequence, the following sets of oligodeoxyribonucleotides were synthesized:

```
              T
       A  C   A  T      T
5'  CA GG   CA AA  TGGGA 3'
    G  A    G  C      C
          G
```

The amino-terminal amino acid sequence of a 10 kD band sometimes present in the preparation was also determined, but not used for the construction of an oligodeoxyribonucleotide probe. The sequence obtained is:

Met-Leu-His-Ile-Leu-X-Gln-Gly-Thr-Pro-Phe-Glu-Ile-Gly-Tyr-Glu-His-Gly-Ser-Ala-Ala-Lys-Ala-Val-Ile-Ala.

EXAMPLE 5

Identification of the acyltransferase gene

The DNA of a number of the lambda clones of Example 2 was digested with restriction endonuclease SalI, the fragments separated on a 0.7% agarose gel, transferred to Genescreen Plus and hybridized to the [³²P]-end labelled oligonucleotide mixtures of Example 4. The clones giving a positive signal were mapped by restriction analysis using standard methods. Two representative physical maps derived for the recombinant phages, lambda B21 and lambda G2, are shown in Figure 2. The oligodeoxyribonucleotide mixture hybridized specifically to the EcoRI/HindIII subfragment indicated on the map. This and the adjacent fragments were recloned in pTZ 18/19 (United States Biochemical Corporation) and subjected to nucleotide sequence analysis. The determined sequence and the deduced amino acid sequence are shown in Figure 3.

The amino-terminal amino acid sequence of a 10 kD band also present in the preparation was determined and found to correspond to a DNA sequence upstream of the 29 kD sequence. Therefore, AT is probably synthesized as a 40 kD protein. This notion is confirmed by the length of the AT messenger, which was demonstrated to be 1500 bases (similar to the isopenicillin N synthetase mRNA which encodes a 38 kD protein), thus allowing for 3' and 5' untranslated regions of 100 bases.

The amino acid sequences of the 29 kD (which has been extended to Thr-Thr-Ala-Tyr-Cys-Gln-Leu-Pro-Asp-Gly-Ala-Leu-Gln-Gly-Gln-Asn-Trp-Asp-Phe-Phe-Ser-Ala-Thr-Lys-Gln-Ala) and 10 kD proteins revealed the presence of two introns. A third intron is postulated on the basis of the gross amino acid composition of the 10 kD protein (97 residues) and on the consensus sequence for its boundaries (D.J. Ballance, Yeast 2 (1986) pp. 229-236). The presence of this third intron was confirmed by primer extension and Northern blot hybridization using oligonucleotide probes from coding and non-coding regions.

EXAMPLE 6

## Construction of pPS47

The phosphoglycerate kinase (pgk) gene was isolated from a Penicillium genomic library by standard methods (Maniatis; Example 1), using the corresponding yeast gene (Hitzeman et al., vide supra) as a hybridization probe. The pgk promoter region is specified in part by the sequence shown in Figure 5, which is located directly upstream of the pgk coding region.

The P. chrysogenum pgk promoter was cloned into pTZ18R as a 1.5 kb HindIII fragment and a clone having the desired orientation was selected.

Subsequently, the phleomycin resistance gene was cloned into the BamHI site of the polylinker of this clone as a 1.0 kb BamHI plus BglII fragment, isolated from pUT702 (Cayla, Toulouse Cedex, France). The pgk promoter was fused in frame to the phleomycin resistance gene, by looping out the sequence to be deleted using an oligonucleotide with the sequence:

5´-GGA ACG GCA CTG GTC AAC TTG GCC ATG GTG GGT AGT TAA TGG TAT G-3´

Moreover, this oligonucleotide introduces an NcoI site at the position of the ATG (underlined).

## EXAMPLE 7

## Construction of a transformation vector with a high transformation efficiency (pPS 54).

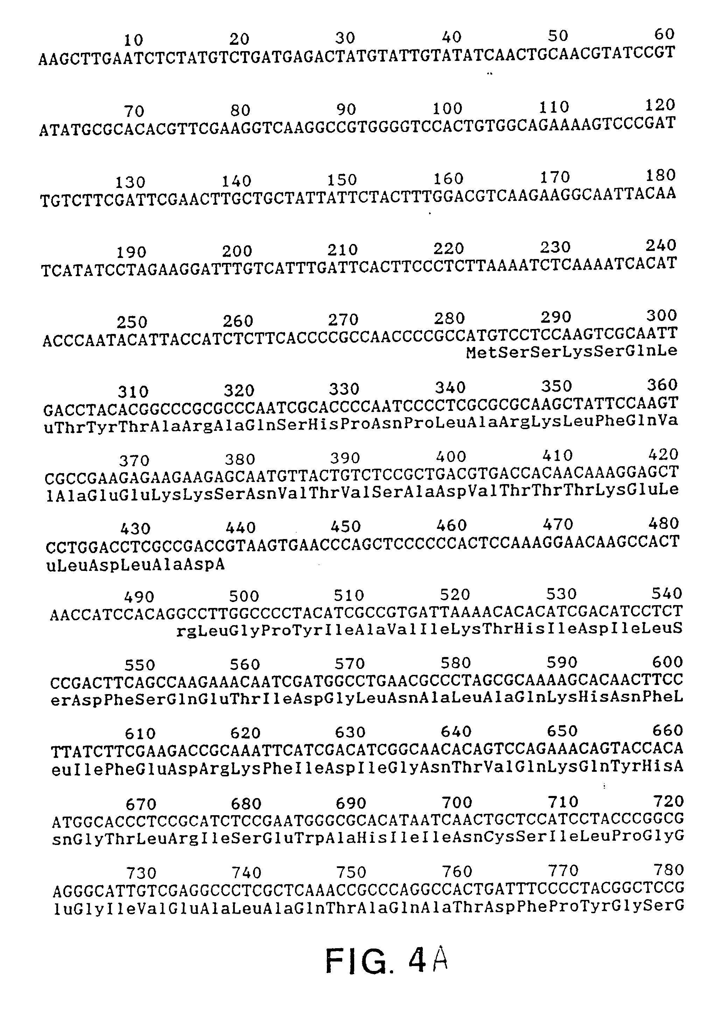

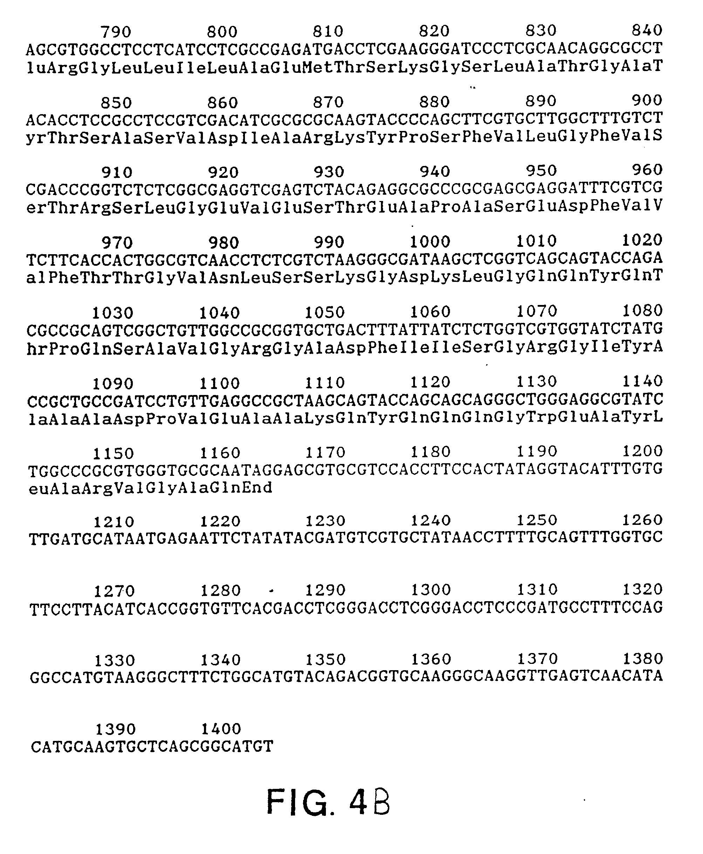

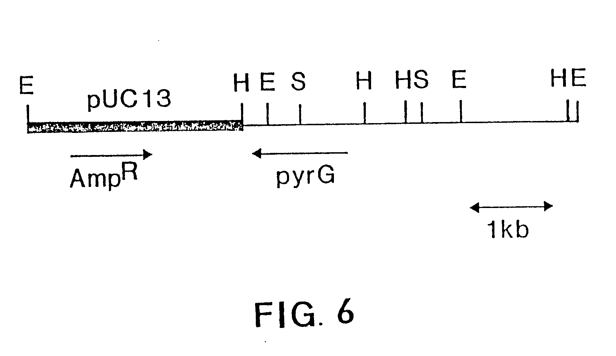

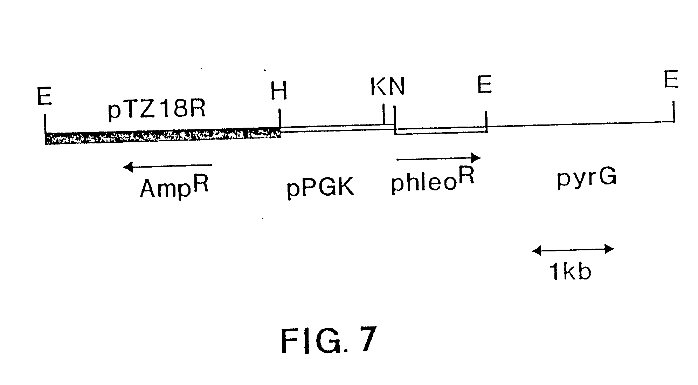

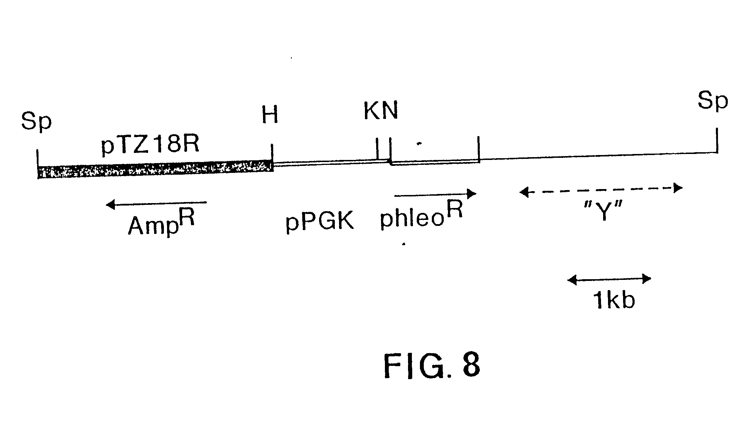

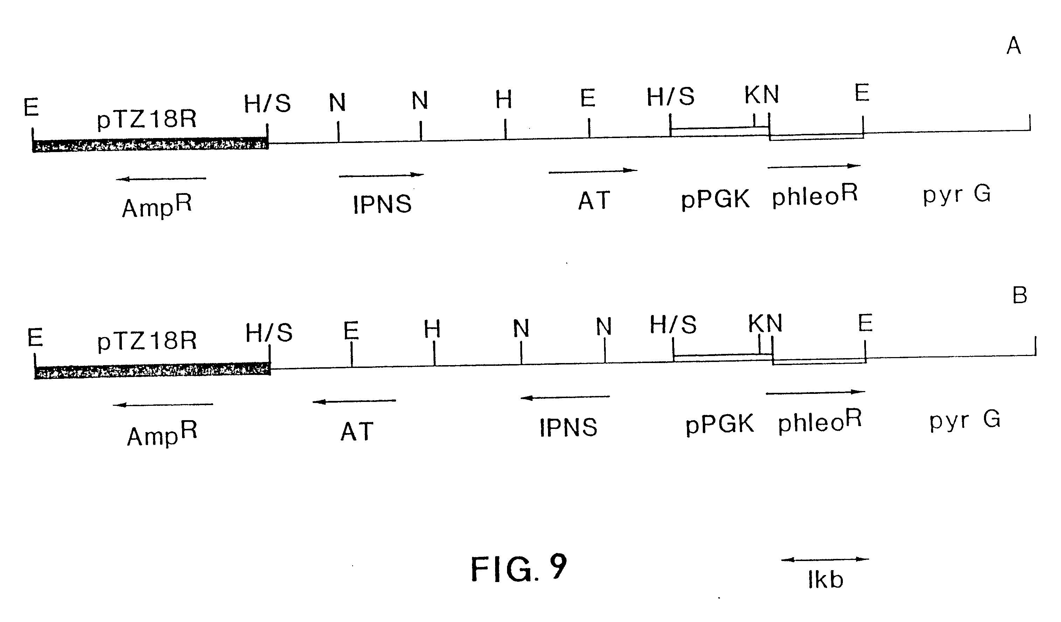

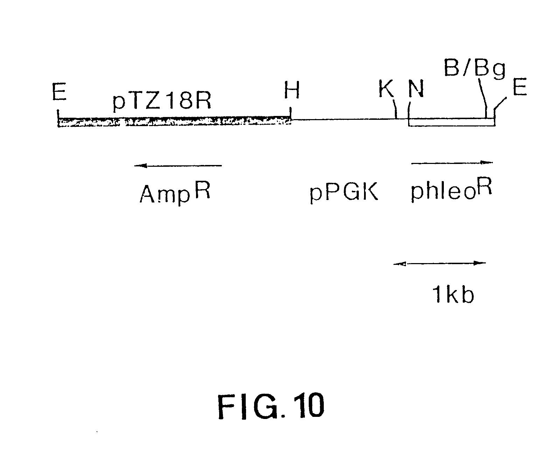

In order to obtain a transformation frequency of P. chrysogenum that is sufficiently high to allow introduction of genes by transformation or cotransformation with the aim of complementing or amplifying non-selectable genes involved in $\beta$-lactam biosynthesis, it is desirable to include in the transformation vector a transformation-enhancing sequence (cf. ans in Aspergillus D.J. Ballance and G. Turner, Gene 36 - (1985) pp. 321-331). Surprisingly, a transformation-stimulating sequence which is functional in P. chrysogenum is present on a DNA fragment containing the P. chrysogenum pyr G gene. Part of this 2.4 kb EcoRI fragment is specified by the nucleotide sequence shown in Figure 4. This DNA fragment forms part of a 4 kb Sau3A partial fragment, cloned in the BamHI site of plasmid pUC 13 (J. Messing, in Meth. Enzymol. 101 (Acad. Press, 1983) p. 20 ff.). This plasmid is referred to as pUC13::pyrG hereinafter (see EP-A-260762 and Figure 6).

The 2.4 kb EcoRI fragment was included in a plasmid (pPS47) containing the phleomycin-resistance gene of Streptoalloteichus hindustanus under the control of the promoter of the phosphoglycerate kinase (pgk) gene from P. chrysogenum. The resulting construct is pPS 54.

The stimulatory effect of the pyrG fragment on the frequency of transformation is shown in Table 1 below:

Table 1

| plasmid | transformants/μg DNA |
|---|---|
| pPS 47 (phleo<sup>R</sup>) | 37 |
| pPS 54 (phleo<sup>R</sup>, pyrG) | 186 |

## EXAMPLE 8

## Biological and biochemical verification of the identity of the AT clones.

The identity of the AT clones was biologically verified by complementation of an acyltransferase-negative mutant of P. chrysogenum Wis 54 - 1255, npe 8.

2 x 107 protoplasts of an uracil-requiring derivative of strain Wis 54-1255 npe 8, Wis 54 - 1255 npe 8 pyrG (CBS 512.88), were cotransformed with a mixture of 5 μg of the selective plasmid pUC 13:: pyrG and 15 μg of lambda B21 DNA as described previously (EP-A-260762).

Several hundreds of transformants were obtained, of which the conidia were collected and plated onto the complex production medium of Example 1 at a density of 1-10 colonies per petri dish. After 3 days incubation at 25°C, the plates were overlayered with a spore suspension of a penicillin-sensitive Bacillus subtilis indicator strain and incubated overnight at 30°C to determine the size of the inhibition zones in the bacterial lawn.

Most (75%) of the transformants showed very small haloes, similar in size to the penicillin non-producing recipient stain npe 8 pyrG. The remaining 25% showed large inhibition zones comparable to those of the wild-type strain, Wis 54-1255. It was concluded that the former class had received only the selective plasmid pUC 13::pyrG, whereas the latter had received both pUC 13:: pyrG and lambda B21, which restores penicillin productivity.

For several transformant clones from both groups, the level of AT-activity in cell-free extracts was determined as follows: Mycelia were collected after two days growth as described in Example 3, washed, frozen in liquid nitrogen and pulverized. For each assay, 2.5 grams of ground mycelium was suspended in 50 mM potassium phosphate buffer (pH 8.0) containing 5 mM dithiothreitol and 5 mM EDTA (final volume 12.5 ml) and stirred for 25 minutes. The cell-free extract was obtained by centrifugation of the suspension (5 minutes at 1000 x g).

AT activity was assayed by incubating 2 ml of cell-free extract with 0.1 ml dithiothreitol (10 mg/ml), 0.2 ml 6-aminopenicillanic acid (10 mg/ml) and 0.2 ml phenylacetylcoenzyme A solution (20 mg/ml) at 25°C.

After 15 or 30 minutes, the reaction was stopped by adding an equal volume of methanol and the sample centrifuged (20 minutes at 5000 x g). The supernatant was then assayed for penicillin G formed by chromatographic (HPLC) methods known in the art. The results of a typical experiment are shown in Table 2 below. These data show that in transformed strains (3) and (4) the level of AT activity is increased 2-3 fold over that of the wild-type (5), consistent with the increased gene dosage.

The IPNS plus AT cluster was subcloned into pPS54, yielding PGJ01 A and B. A SalI fragment of 5 kb was made blunt by the action of T4 DNA polymerase and ligated into the unique HindIII site of pPS54, after treatment of this vector with T4 DNA polymerase.

Table 2

| | STRAIN | TRANSFORMED WITH: | HALO: | UNITS* PEN-G FORMED PER MG PROTEIN, | | NUMBER OF AT COPIES AS ESTIMATED BY SOUTHERN HYBRIDIZATION |
|---|---|---|---|---|---|---|
| | | | | AFTER 15 minutes | AFTER 30 minutes | |
| 1) | Wis 54-1255 npe 8 pyrG | pUC 13::pyrG | - | passes test | 0.9 | 1** |
| 2) | idem | pUC 13::pyrG plus lambda B21 | - | 1.7 | 1.1 | 1** |
| 3) | idem | idem | + | 11.9 | 9.5 | > 1 |
| 4) | idem | idem | + | 10.8 | 7.0 | > 1 |
| 5) | Wis 54-1255 | not transformed | + | 4.5 | 2.7 | 1 |

* relative AT activity in extract.
** inactive by mutation

EP 0 354 624 A2

## EXAMPLE 9

Increased penicillin production in a host strain transformed with the cryptic gene Y.

To show the effect of the genes identified herein as involved in penicillin production, the gene dosage of one of these genes was increased in a Penicillium host strain. To this end the gene "Y", contained in lambda clones B9, L5 and G5, was subcloned as a 3.0 kb BamHI plus SphI fragment into pPS47. The resulting construct, pRH05 was transformed to P. chrysogenum Wis 54-1255 (ATCC 28089) and phleomycin resistant clones were isolated. Several clones were tested for penicillin production in shake flasks.

The results obtained for one transformant isolated are shown in Table 3 below.

Table 3

| strain | relative production of penicillin |
|---|---|
| Wis 54-1255 | 100 |
| Wis 54-1255::pRH05 | 122 |

The increased gene dosage of gene Y in the transformant, as compared to the untransformed host, was confirmed by Southern blot analysis. Hence the increased gene dosage of gene Y, a cryptic gene, isolated by the method of the invention, results in a substantial increase in penicillin production.

The transcript size for gene Y has been determined by Northern blot hybridization: the transcript is about 1.0 kb long.

## Claims

1. A method for isolating DNA-fragments expressed during the metabolism of microorganisms, and which are directly or indirectly essential for the production of a secondary metabolite in this microorganism, preferably a bacterium or fungus, said method comprising:

(a) screening a DNA library prepared from said microorganism with probes obtained from mRNA or DNA derived from a first culture of said microorganism, producing said secondary metabolite;

(b) screening said DNA library with probes obtained from mRNA or DNA derived from a second culture of said microorganism or a mutant thereof, lacking the production of said secondary metabolite; and

(c) identifying fragments comprising genes transcribed in said first culture which are not substantially transcribed in said second culture.

2. A method for preparing a DNA construct comprising:

(a) isolating fragments expressed during the production of a secondary metabolite according to claim 1; and

(b) subcloning the isolated fragments to form a DNA construct which contains the gene(s) of interest and all necessary transcriptional and translational elements and optionally a selection marker.

3. A method for obtaining or enhancing the production of a secondary metabolite in a microbial host comprising:

(a) prepararing DNA constructs according to claim 3;

(b) transforming a candidate host with these DNA constructs;

(c) cloning the resulting transformants; and

(d) identifying clones producing said secondary metabolite at a higher level than said candidate host.

4. A method according to Claim 1, wherein said first and second cultures have the ability to produce $\beta$-lactam antibiotics.

5. A method according to Claim 1, wherein said first and second cultures are Penicillium, Aspergillus, Acremonium, Flavobacterium or Actinomycetes.

6. A vector comprising a sequence encoding a gene directly or indirectly involved in the biosynthetic pathway leading to a secondary metabolite, optionally a marker for selection in a host producing said secondary metabolite and optionally a sequence for enhancing transformation efficiency of said vector in said host, wherein said gene is selected from the group consisting of the acyltransferase gene, cryptic gene Y, and the cryptic genes contained entirely or partly within the phages L12, K9, C12, P3, K11, B13, B20, G3, G1, L10, K16 and B23.

7. A vector comprising a sequence encoding acyltransferase of a structure as given in Figure 3.

8. A vector according to Claim 6, comprising a sequence encoding acyltransferase, which has been modified by insertion mutagenesis, deletion mutagenesis, a combination of insertion and deletion mutagenesis, or site specific mutagenesis of the DNA sequence specified in Figure 3.

9. A vector according to Claim 6, wherein said gene comprises another than the endogenous promoter for transcription.

10. A transformed host comprising a DNA construct prepared with the method of Claim 2.

11. A transformed host comprising as a result of transformation a copy of a sequence comprising a gene functional in said host and encoding a protein directly or indirectly involved in the biosynthetic pathway leading to a secondary metabolite resulting in increased production of said secondary metabolite, wherein said gene is selected from the group consisting of the acyltransferase gene, cryptic gene Y, and the cryptic genes contained entirely or partly within the phages L12, K9, C12, P3, K11, B13, B20, G3, G1, L10, K16 and B23.

12. A novel strain obtained by strain improvement procedures other than DNA mediated transformation using a transformed host comprising a vector according to Claim 6 or a transformed host according to Claim 10 or 11.

13. A transformed host according to Claim 10 or 11, wherein said host is capable of producing $\beta$-lactam antibiotics.

14. A transformed host according to Claim 10 or 11, whereinsaid host is a Penicillium, Aspergillus, Acremonium, Flavobacterium or Actinomycetes.

15. A transformed host according to Claim 10, wherein said secondary metabolite is a $\beta$-lactam.

16. A transformed host according to Claim 10, wherein said $\beta$-lactam is penicillin.

17. A transformed host according to Claim 14, wherein said host is the species Penicillium chrysogenum or Acremonium chrysogenum or E. coli.

18. A transformed host according to Claim 14, wherein said gene is under the transcriptional initiation regulation of other than the wild-type transcriptional initiation regulatory region.

19. A DNA construct comprising the P. chrysogenum phosphoglycerate kinase gene promoter and genes, prepared according to the method of Claim 2, under the transcriptional control of said promoter.

20. A DNA construct prepared according to Claim 2, wherein the selection marker is a phleomycin resistance gene, which is expressed under the transcriptional control of the phosphoglycerate kinase gene promoter.

21. A recombinant DNA construct comprising the promoter and translational activating sequence of the acyltransferase gene of Penicillium chrysogenum.

22. A recombinant DNA construct comprising the 3'-regulatory sequences of the acyltransferase gene of Penicillium chrysogenum.

23. A recombinant DNA construct that encodes acyltransferase activity and that can be identified by using a DNA compound of Figure 3 as a probe.

24. A plasmid selected from the group consisting of PGJ01 A or B, or pRH05.

25. A DNA construct comprising a transformation enhancing sequence comprising the sequence of Figure 4 joined to another than the wild-type sequence.

26. A DNA sequence according to Claim 25, wherein said other than wild-type sequence comprises a gene encoding an enzymatic activity or Penicillium.

27. A method for providing improved yields of an antibiotic secondary metabolite comprising:

(a) growing a transformed host comprising an extra copy of a sequence comprising a gene, isolated according to the method of Claim 1 or 2, encoding a protein directly or indirectly involved in the biosynthetic pathway of the production of an antibiotic secondary metabolite resulting in the enhanced production of said antibiotic; and

(b) isolating the resulting antibiotic product.

28. A method according to Claim 27, wherein said antibiotic is a β-lactam.

29. A method according to Claim 28, wherein said host is a Penicillium Aspergillus, Acremonium, Flavobacterium or Actinomycetes.

30. A method according to Claim 29, wherein said host is Penicillium chrysogenum.

31. A method according to Claim 27, wherein said gene encodes acyltransferase.

32. A method according to Claim 27, wherein said gene encodes a protein of a structure as given in Fig. 3.

33. A method according to Claim 27, wherein said gene is cryptic gene Y.

EP 0 354 624 A2

L-α-aminoadipic acid    L-cysteine    L-valine

ACV-SYNTHETASE
(ACVS)

δ-(L-α-aminoadipyl)-L-cysteinyl-D-valine

ISOPENICILLIN N SYNTHETASE
(IPNS; cyclase)

isopenicillin N (IPN)

ACYL-CoA:IPN
ACYLTRANSFERASE
(AT)

6-aminopenicillanic acid
(6-APA)

phenylacetic or
phenoxyacetic acid

penicillin-G or V

FIG. 1

FIG. 2

EP 0 354 624 A2

AAGCTTTCAGGCAACCTAGGCAACCCAATAGGAACCAAGTGATAGGCCCACCTGCTTTCT

ATCTAGTCTGGACGGTTGCTATTGGCTCGATCATTGTTTACCATCCCGGCAAAAAGCTCT

ACAGAGTTGTGCTATTTCTATTCCTGTCTTGGCATGTCCAGGCTGGCTGTTATCGCCTCC

GTGGTGAACCCTCTTCATGCAAGAGGTCAGTCAATAATGCGCTTCACCGTTCTCGACGAA

ACTTGGCATCCATGCTCAATCCAGCTCCTCGGCAAGACTAGGCGGATGCAGCAGGGATAC

TCGAGGTGCCCCAGTTGATGTCCCATCAGTGTCATGCTATGGTCCCAGATTGGTGGCTAC

GGCCAATATAAATCTCAGCATGCAGTTCCGCCTGCATGATCATCCCCAGGACGCGTTTGT

CATCTCCGTCAGCCAGGTCTCAGTTGTTTACCCATCTTCCGACCCGCAGCAGAAATGCTT
                                                        MetLeu

CACATCCTCTGTCAAGGCACTCCCTTTGAAGTAAGTGCTGCACTGAATACCAGATTTTTT
HisIleLeuCysGlnGlyThrProPheGlu

CCTTCTGAATCTTCCGAGTTCTGACCTGATCCAGATCGGCTACGAACATGGCTCTGCTGC
                                ILeGlyTyrGluHisGlySerAlaAl

CAAAGCCGTGATAGCCAGAAGCATTGACTTCGCCGTCGATCTCATCCGAGGGAAAACGAA
aLysAlaValIleAlaArgSerIleAspPheAlaValAspLeuIleArgGlyLysThrLy

GAAGACGGACGAAGAGCTTAAACAGGTACTCTCGCAACTGGGGCGCGTGATCGAGGAAAG
sLysThrAspGluGluLeuLysGlnValLeuSerGlnLeuGlyArgValIleGluGluAr

ATGGCCCAAATACTACGAGGAGATTCGCGGTGAGTGCCACTTCGGTCTTTCCTACATTTT
gTrpProLysTyrTyrGluGluIleArgG

# FIG. 3A

```
         790        800        810        820        830        840
CTGCACCAATGCTGACCGATGACCCCCGAAAAACCAGGTATTGCAAAGGGCGCTGAACGC
                                   lyIleAlaLysGlyAlaGluArg


         850        860        870        880        890        900
GATGTCTCCGAGATTGTCATGCTTAATACCCGCACGGAATTTGCATACGGGCTCAAGGCA
AspValSerGluIleValMetLeuAsnThrArgThrGluPheAlaTyrGlyLeuLysAla


         910        920        930        940        950        960
GCCCGTGATGGCTGCACCACTGCCTATTGTCAACTTCCAAATGGAGCCCTCCAGGGCCAA
AlaArgAspGlyCysThrThrAlaTyrCysGlnLeuProAsnGlyAlaLeuGlnGlyGln


         970        980        990       1000       1010       1020
AACTGGGATGTACGTTAAGAGATTTTACCTCCTCATTTTATTCCATCGAATTTGCGCCGA
AsnTrpAsp


        1030       1040       1050       1060       1070       1080
CTAATTTGGTTGTTCAAGTTCTTTTCTGCCACCAAAGAGAACCTGATCCGGTTAACGATC
                 PhePheSerAlaThrLysGluAsnLeuIleArgLeuThrIle


        1090       1100       1110       1120       1130       1140
CGTCAGGCCGGACTTCCCACCATCAAATTCATAACCGAGGCTGGAATCATCGGGAAGGTT
ArgGlnAlaGlyLeuProThrIleLysPheIleThrGluAlaGlyIleIleGlyLysVal


        1150       1160       1170       1180       1190       1200
GGATTTAACAGTGCGGGGGTCGCCGTCAATTACAACGCCCTTCACCTTCAGGGTCTTCGA
GlyPheAsnSerAlaGlyValAlaValAsnTyrAsnAlaLeuHisLeuGlnGlyLeuArg


        1210       1220       1230       1240       1250       1260
CCCACCGGAGTTCCTTCGCATATTGCCCTCCGCATAGCGCTCGAAAGCACTTCTCCTTCC
ProThrGlyValProSerHisIleAlaLeuArgIleAlaLeuGluSerThrSerProSer


        1270       1280       1290       1300       1310       1320
CAGGCCTATGACCGGATCGTGGAGCAAGGCGGAATGGCCGCCAGCGCTTTTATCATGGTG
GlnAlaTyrAspArgIleValGluGlnGlyGlyMetAlaAlaSerAlaPheIleMetVal


        1330       1340       1350       1360       1370       1380
GGCAATGGGCACGAGGCATTTGGTTTGGAATTCTCCCCCACCAGCATCCGAAAGCAGGTG
GlyAsnGlyHisGluAlaPheGlyLeuGluPheSerProThrSerIleArgLysGlnVal


        1390       1400       1410       1420       1430       1440
CTCGACGCGAATGGTAGGATGGTGCACACCAACCACTGCTTGCTTCAGCACGGCAAAAAT
LeuAspAlaAsnGlyArgMetValHisThrAsnHisCysLeuLeuGlnHisGlyLysAsn


        1450       1460       1470       1480       1490       1500
GAGAAAGAGCTCGATCCCTTACCGGACTCATGGAATCGCCACCAGCGTATGGAGTTCCTC
GluLysGluLeuAspProLeuProAspSerTrpAsnArgHisGlnArgMetGluPheLeu


        1510       1520       1530       1540       1550       1560
CTCGACGGGTTCGACGGCACCAAACAGGCATTTGCCCAGCTCTGGGCCGACGAAGACAAT
LeuAspGlyPheAspGlyThrLysGlnAlaPheAlaGlnLeuTrpAlaAspGluAspAsn


        1570       1580       1590       1600       1610       1620
TATCCCTTTAGCATCTGCCGCGCTTACGAGGAGGGCAAGAGCAGAGGCGCGACTCTGTTC
TyrProPheSerIleCysArgAlaTyrGluGluGlyLysSerArgGlyAlaThrLeuPhe
```

# FIG. 3B

```
              1630      1640      1650      1660      1670      1680
AATATCATCTACGACCATGCCCGTAGAGAGGCAACGGTGCGGCTTGGCCGGCCGACCAAC
AsnIleIleTyrAspHisAlaArgArgGluAlaThrValArgLeuGlyArgProThrAsn

              1690      1700      1710      1720      1730      1740
CCTGATGAGATGTTTGTCATGCGGTTTGACGAGGAGGACGAGAGGTCTGCGCTCAACGCC
ProAspGluMetPheValMetArgPheAspGluGluAspGluArgSerAlaLeuAsnAla

              1750      1760      1770      1780      1790      1800
AGGCTTTGAAGGCTCTTCATGACGAGCCAATGCATCTTTTGTATGTAGCTTCAACCGACT
ArgLeuEnd

              1810      1820      1830      1840      1850      1860
CCGTCTTCACTTCTTCGCCCGCACTGCCTACCGTTTGTACCATCTGACTCATATAAATGT

              1870      1880      1890      1900      1910      1920
CTAGCCCCTACCTACACTATACCTAAGGGAGAGAAGCGTAGAGTGATTAACGTACGGGCC

              1930      1940      1950      1960      1970      1980
TATAGTACCCCGATCTCTAGATAGAACATTTAGTAGAGATTAGGATGCCTAACTAATTTA

              1990      2000      2010      2020      2030      2040
ACTTGAGCATTGTCCCGTTCATATTGATTTTCAGTCCATTATACACTCTTAATCGTTTCC

              2050      2060      2070      2080      2090      2100
CGGTAGAAGCCTGATATATACGACCATAGGGTGTGGAGAACAGGGCTTCCCGTCTGCTTG

              2110      2120      2130      2140      2150      2160
GCCGTACTTAAGCTATATATTCTACACGGCCAATACTCAATGTGCCCTTAGCACCTAAGC

              2170      2180      2190      2200      2210      2220
GGCACTCTAGGGTAAGTGCGGGTGATATAGGTGAGAAGTCTTAAGACTGAAGACAGGATA

              2230      2240      2250      2260      2270      2280
TCACGCGTTACCCTGCACCGTACCTACTACCTTCAATATCAACTCTTTCAGGATGGACAG


GGTCGAC
```

# FIG. 3C

AAGCTTGAATCTCTATGTCTGATGAGACTATGTATTGTATATCAACTGCAACGTATCCGT

ATATGCGCACACGTTCGAAGGTCAAGGCCGTGGGGTCCACTGTGGCAGAAAAGTCCCGAT

TGTCTTCGATTCGAACTTGCTGCTATTATTCTACTTTGGACGTCAAGAAGGCAATTACAA

TCATATCCTAGAAGGATTTGTCATTTGATTCACTTCCCTCTTAAAATCTCAAAATCACAT

ACCCAATACATTACCATCTCTTCACCCCGCCAACCCCGCCATGTCCTCCAAGTCGCAATT
                                         MetSerSerLysSerGlnLe

GACCTACACGGCCCGCGCCCAATCGCACCCCAATCCCCTCGCGCGCAAGCTATTCCAAGT
uThrTyrThrAlaArgAlaGlnSerHisProAsnProLeuAlaArgLysLeuPheGlnVa

CGCCGAAGAGAAGAAGAGCAATGTTACTGTCTCCGCTGACGTGACCACAACAAAGGAGCT
lAlaGluGluLysLysSerAsnValThrValSerAlaAspValThrThrThrLysGluLe

CCTGGACCTCGCCGACCGTAAGTGAACCCAGCTCCCCCCACTCCAAAGGAACAAGCCACT
uLeuAspLeuAlaAspA

AACCATCCACAGGCCTTGGCCCCTACATCGCCGTGATTAAAACACACATCGACATCCTCT
        rgLeuGlyProTyrIleAlaValIleLysThrHisIleAspIleLeuS

CCGACTTCAGCCAAGAAACAATCGATGGCCTGAACGCCCTAGCGCAAAAGCACAACTTCC
erAspPheSerGlnGluThrIleAspGlyLeuAsnAlaLeuAlaGlnLysHisAsnPheL

TTATCTTCGAAGACCGCAAATTCATCGACATCGGCAACACAGTCCAGAAACAGTACCACA
euIlePheGluAspArgLysPheIleAspIleGlyAsnThrValGlnLysGlnTyrHisA

ATGGCACCCTCCGCATCTCCGAATGGGCGCACATAATCAACTGCTCCATCCTACCCGGCG
snGlyThrLeuArgIleSerGluTrpAlaHisIleIleAsnCysSerIleLeuProGlyG

AGGGCATTGTCGAGGCCCTCGCTCAAACCGCCCAGGCCACTGATTTCCCCTACGGCTCCG
luGlyIleValGluAlaLeuAlaGlnThrAlaGlnAlaThrAspPheProTyrGlySerG

# FIG. 4A

```
          790       800       810       820       830       840
AGCGTGGCCTCCTCATCCTCGCCGAGATGACCTCGAAGGGATCCCTCGCAACAGGCGCCT
luArgGlyLeuLeuIleLeuAlaGluMetThrSerLysGlySerLeuAlaThrGlyAlaT


          850       860       870       880       890       900
ACACCTCCGCCTCCGTCGACATCGCGCGCAAGTACCCCAGCTTCGTGCTTGGCTTTGTCT
yrThrSerAlaSerValAspIleAlaArgLysTyrProSerPheValLeuGlyPheValS


          910       920       930       940       950       960
CGACCCGGTCTCTCGGCGAGGTCGAGTCTACAGAGGCGCCCGCGAGCGAGGATTTCGTCG
erThrArgSerLeuGlyGluValGluSerThrGluAlaProAlaSerGluAspPheValV


          970       980       990      1000      1010      1020
TCTTCACCACTGGCGTCAACCTCTCGTCTAAGGGCGATAAGCTCGGTCAGCAGTACCAGA
alPheThrThrGlyValAsnLeuSerSerLysGlyAspLysLeuGlyGlnGlnTyrGlnT


         1030      1040      1050      1060      1070      1080
CGCCGCAGTCGGCTGTTGGCCGCGGTGCTGACTTTATTATCTCTGGTCGTGGTATCTATG
hrProGlnSerAlaValGlyArgGlyAlaAspPheIleIleSerGlyArgGlyIleTyrA


         1090      1100      1110      1120      1130      1140
CCGCTGCCGATCCTGTTGAGGCCGCTAAGCAGTACCAGCAGCAGGGCTGGGAGGCGTATC
laAlaAlaAspProValGluAlaAlaLysGlnTyrGlnGlnGlnGlyTrpGluAlaTyrL


         1150      1160      1170      1180      1190      1200
TGGCCCGCGTGGGTGCGCAATAGGAGCGTGCGTCCACCTTCCACTATAGGTACATTTGTG
euAlaArgValGlyAlaGlnEnd


         1210      1220      1230      1240      1250      1260
TTGATGCATAATGAGAATTCTATATACGATGTCGTGCTATAACCTTTTGCAGTTTGGTGC


         1270      1280     1290      1300      1310      1320
TTCCTTACATCACCGGTGTTCACGACCTCGGGACCTCGGGACCTCCCGATGCCTTTCCAG


         1330      1340      1350      1360      1370      1380
GGCCATGTAAGGGCTTTCTGGCATGTACAGACGGTGCAAGGGCAAGGTTGAGTCAACATA


         1390      1400
CATGCAAGTGCTCAGCGGCATGT
```

# FIG. 4B

```
         10        20        30        40        50        60
CTATGAATTCCCATCAGTAGAGGCCAATTCCCGAGAATTTCCCGATTCCGAGGCTAAAGA

         70        80        90       100       110       120
GGCTAAAGAGGACCTCCATGGCCCGGCCTTCCCCGTATCAACGCGGGCAGCTCTGACATC

        130       140       150       160       170       180
AGTCCCGCGTCAGCCCGATCAGTTCTCCGGGATACTCGGTACGGCGTCTGAGGTACTGCT

        190       200       210       220       230       240
TTTATAGATATACCTAGGAAAAGAGGGATTGCATCATACTTCGAGCAAGTACCTTATTCC

        250       260       270       280       290       300
GTCGAATACATCATCGGGAGGTACCTGGGTTGCTACGGTTGCGACCTCTTCACCGGCCTG

        310       320       330       340       350       360
CCCCACCAAAACTGACCCCTCCACTCTTTACTGATCGCGACTCACCGTGGCCTGATTAGT

        370       380       390       400       410       420
TCTTTCTCCTTCTTCTATCCCTTTTCTTGCTCCTTTCACCATTGTCATACCATTAACTAC

CCACA
```

# FIG. 5

FIG. 6

FIG. 7

EP 0 354 624 A2

Sp        pTZ18R        H        KN        Sp

AmpR        pPGK    phleoR        "Y"

1kb

FIG. 8

FIG. 9

1kb

EP 0 354 624 A2

E   pTZ18R   H   K   N   B/Bg   E

AmpR   pPGK   phleoR

1kb

# FIG. 10